## Europäisches Patentamt

## European Patent Office

## Office européen des brevets

(19)

(11) EP 0 872 733 A1

(12) **EUROPÄISCHE PATENTANMELDUNG**

(43) Veröffentlichungstag:
21.10.1998 Patentblatt 1998/43

(21) Anmeldenummer: 98106612.9

(22) Anmeldetag: 09.04.1998

(51) Int. Cl.⁶: **G01N 33/483**, G01N 21/64,
G01N 21/55, G01N 33/543,
G01N 33/533, G01N 33/553

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 14.04.1997 DE 19715483

(71) Anmelder:
BOEHRINGER MANNHEIM GMBH
68305 Mannheim (DE)

(72) Erfinder:
• Herrmann, Rupert, Dr.
82362 Weilheim (DE)
• Sluka, Peter, Dr.
82362 Weilheim (DE)
• Knoll, Wolfgang, Prof. Dr.
55124 Mainz (DE)
• Liebermann, Thorsten
64380 Rossdorf (DE)

(74) Vertreter:
Weiss, Wolfgang, Dipl.-Chem. Dr. et al
Postfach 86 08 20
81635 München (DE)

(54) **Methode zur gleichzeitigen Bestimmung von biomolekularen Wechselwirkungen mittels Plasmonenresonanz und Fluoreszenzdetektion**

(57) Es wird ein Verfahren zum Nachweis eines Analyten beschrieben, welches dadurch gekennzeichnet ist, daß man die Bindung des Analyten an eine Festphae durch unabhängige Auswertung der Signale aus einer Plasmonenresonanz- und einer Fluoreszenzmessung bestimmt.

**Beschreibung**

Die Erfindung betrifft ein Verfahren zum Nachweis eines Analyten, wobei man zum Nachweis eine Kombination von Plasmonenresonanz- und Fluoreszenzmessung durchführt, sowie eine Vorrichtung, welche zur Durchführung dieses Verfahrens geeignet ist.

Die Oberflächenplasmonenspektroskopie wird zur Bestimmung optischer Schichtdicken von adsorbierten Substanzen an Festphasen eingesetzt. Für diese Nachweismethode wird keine Markierung benötigt und es ist möglich, sogenannte Echtzeit-Messungen durchzuführen, mit denen die Kinetik von Reaktionen biomolekularen Wechselwirkungen und Adsorptionsvorgängen bestimmt werden kann. Nachteile der Oberflächenplasmonenresonanz sind die geringe Sensitivität mit einer Nachweisgrenze von etwa $10^{-10}$ mol/l sowie Schwierigkeiten und Beschränkungen beim Vermessen von relativ kleinen Molekülen.

Die Fluoreszenzdetektion unter Verwendung von Fluoreszenzmarkierungen findet z. B. in der Biochemie und auch in der Diagnostik Anwendung. Die Anregung von Fluoreszenz an Oberflächen kann beispielsweise über eine externe Lichtquelle oder auch über sogenanntes evaneszentes Licht erfolgen. Eine solche Fluoreszenzanregung über evaneszentes Licht erfolgt beispielsweise beim e-wave-Konzept, bei dem das in einem Lichtleiter geführte Licht die auf der Oberfläche dieses Lichtleiters immobilisierten Markierungen zur Fluoreszenz anregt (W.F. Love et al. Biosensors and Bioelectronics 7 (1992), 39-48). Es ist möglich, Plasmonenlicht als evaneszentes Licht zur Anregung von an der Oberfläche gebundenen Fluoreszenzmarkierungen zu verwenden. Die Intensität des evaneszenten Lichtes ist dabei um ein Vielfaches höher als die Intensität des eingestrahlten Lichtes, so dass eine deutliche Steigerung der Empfindlichkeit der Fluoreszenzdetektion herbeigeführt werden kann. Es wird also der Feldverstärkungseffekt der Oberflächen-Plasmonenresonanz verwendet, um das Fluoreszenzsignal zu erhöhen, wie beispielsweise bei Kitson et al., Journal of Modern Optics, Vol. 43 (3), (1996), 573-582 und EP-0 353 937 beschrieben. Bei den dort beschriebenen Verfahren handelt es sich um sensitive Varianten der Fluoreszenzdetektion, welche jedoch den Nachteil aufweisen, dass keine kinetischen Messungen möglich sind.

Eine Aufgabe der vorliegenden Erfindung war es deshalb, ein Verfahren bereitzustellen, welches es ermöglicht, einen Analyten mit hoher Sensitivität nachzuweisen und das gleichzeitig die Bestimmung von Reaktionskinetiken erlaubt.

Diese Aufgabe wird erfindungsgemäß gelöst durch ein Verfahren zum Nachweis eines Analyten, welches dadurch gekennzeichnet ist, daß man die Bindung des Analyten an eine Festphase durch unabhängige Auswertung der Signale aus einer Plasmonenresonanz- und einer Fluoreszenzmessung bestimmt. Überraschenderweise ist es mit dem erfindungsgemäßen Verfahren möglich, Echtzeit-Messungen durchzuführen, während gleichzeitig eine hohe Sensitivität erreicht wird. Durch die Kombination der beiden Methoden Plasmonenresonanz- und Fluoreszenzdetektion werden erfindungsgemäß zwei unabhängige Meßsignale erhalten.

Ein wesentlicher Vorteil des erfindungsgemäßen Verfahrens besteht darin, dass durch die Plasmonenresonanz, bei der die Schichtdicke bestimmt wird, auch unspezifische Wechselwirkungen der den Analyten enthaltenden Probe mit der Festphase erfaßt werden können, während die Fluoreszenzdetektion ausschließlich die spezifische Wechselwirkung des Analyten mit der Festphase erfaßt. Dadurch ist es möglich, den Anteil an spezifischer und unspezifischer Bindung in einer Probe zu bestimmen und zwischen beiden Bindungsarten zu differenzieren. Darüber hinaus sind mit der Plasmonenresonanz zusätzlich kinetische Messungen möglich, z. B. die Beobachtung der Adsorption über die Zeit, während mit der Fluoreszenzdetektion Endpunktmessungen durchgeführt werden können. Bei der Fluoreszenzdetektion wird dabei eine Sensitivität von $10^{-13}$ bis $10^{-14}$ mol/l erreicht, während bei der Plasmonenresonanz eine Sensitivität von etwa $10^{-10}$ mol/l erreicht wird.

Durch Feldverstärkung durch resonante Einkopplung des einfallenden Lichts im Reflektivitätsminimum der Plasmonenresonanz kann die Sensitivität des Fluoreszenznachweises zusätzlich verbessert werden. Diese Feldverstärkung kann zur Anregung von adsorbierten Farbstoffmolekülen verwendet werden, wodurch eine oberflächensensitive Nachweismethode erhalten wird, da der evaneszente Charakter der Feldstärke eine dominante Anregung oberflächenadsorpierter Fluorophore bewirkt. Da diese Anregung spezifisch nur bei auf der Oberfläche gebundenen Farbstoffmolekülen auftritt, ist ein Abtrennung nicht an die Oberfläche von gebundenem Farbstoff nicht notwendig.

Mit dem erfindungsgemäßen Verfahren können z. B. biomolekulare Wechselwirkungen, Reaktionskinetiken und Adsorptionsvorgänge untersucht werden, aber auch Analyten qualitativ oder/und quantitativ bestimmt werden.

Das erfindungsgemäße Verfahren kann unter Ausnutzung der intrinsischen Fluoreszenz der an der Nachweisreaktion eines Analyten beteiligten Reaktionspartner durchgeführt werden. Bevorzugt wird für den Nachweis jedoch mindestens ein fluoreszenzmarkiertes Reagenz verwendet.

Als Festphase wird für das erfindungsgemäße Verfahren bevorzugt ein optisch durchlässiger Träger, der mit einer Metall- oder Metall/Metalloxidschicht überzogen ist, verwendet. Der optisch durchlässige Träger, beispielsweise aus Glas oder Quarzglas, kann hierzu z.B. mit einer relativ dünnen Metall- oder Metall/Metalloxidschicht bedampft werden. Die Metall/Metalloxidschicht besteht dabei bevorzugt aus einer Metallschicht, welche zuerst auf den optisch durchlässigen Träger aufgedampft wird, und einer Metalloxidschicht, welche anschließend auf die Metallschicht aufgebracht wird. Die Dicke des Überzugs beträgt bevorzugt zwischen 10 nm und 1 µm, besonders bevorzugt zwischen 30 nm und 100 nm. Falls der Träger mit einer Metallschicht bedampft wird, verwendet man bevorzugt Edelmetalle und besonders bevorzugt Gold oder Silber. Bevorzugte Metalloxidschichten umfassen $SiO_2$, $TiO_2$, $Al_2O_3$ und $Ag_2O$.

Auf der Metall- oder Metalloxidschicht befindet sich bevorzugt eine Festphasenbindematrix, über die der Analyt mit der Festphase verknüpft wird. Eine Festphasenbindematrix umfaßt einen Festphasenreaktanten, der eine spezifische Wechselwirkung mit einem Bindepartner, z. B. einen zu bestimmenden Analyten eingehen kann. Der Festphasenreaktant kann kovalent oder adsorptiv über Angergruppen mit der Oberfläche verknüpft sein, gegebenenfalls über Spacermoleküle. Besonders bevorzugt erfolgt die Verknüpfung über eine selbstassemblierende Monoschicht ("self-assembled Monolayer", SAM), bei der eine adsorptive Bindung von Thiol- oder Disulfidgruppen an eine Metalloberfläche erfolgt. Solche selbstassemblierenden Monoschichten sind beispielsweise in DE 40 39 677 beschrieben, deren Inhalt durch Bezugnahme Teil dieser Anmeldung wird. Bevorzugt wird durch Zusatz von Verdünnermolekülen oder durch Behandlung einer Oberfläche mit in hoher Verdünnung vorliegenden Festphasen-Reaktanden eine verdünnte und im wesentlichen lateral homogene Bindeschicht auf der Oberfläche des Trägermaterials gebildet, welche besonders vorteilhafte Bindeeigenschften aufweist. Es ist aber auch möglich, als Festphasenbindematrix herkömmliche Langmuirschichten zu verwenden (Blankenburg et al., Biochemistry 28 (1989) 8214; Ahlers et al., Thin Solid Films 180 (1989) 93-99). Zum Aufbringen auf Metalloxidschichten besonders geeignete Silan-Verbindungen sind in DE 4401450 beschrieben.

Als Lichtquelle für die Plasmonenresonanz und für die Fluoreszenzdetektion wird vorzugsweise ein Laser eingesetzt, welcher monochromatisches Licht zur Verfügung stellt. Es ist jedoch auch möglich, andere Lichtquellen zu verwenden.

Der Abstand zwischen der Festphasenoberfläche und dem zum Fluoreszenznachweis verwendeten Fluorophor beträgt bevorzugt $\geq 5$ nm, besonders bevorzugt $\geq 20$ nm, um eine nachteilige Beeinflussung der abgestrahlten Fluoreszenz durch die Metalloberfläche zu vermeiden. Der Abstand kann z. B. durch Verwendung geeigneter Spacermoleküle erzielt werden. Die Detektion der Plasmonenresonanz kann auf bekannte Weise, z.B. mittels einer Photodiode erfolgen. Die Fluoreszenzdetektion kann ebenfalls auf jede bekannte Weise, z.B. mit einem Photomultiplier durchgeführt werden. Durch Verwendung entsprechender Vorrichtungen, wie beispielsweise einer Mikroskopoptik zur Aufweitung und Parallelisierung des Laserstrahls und Detektion über Scanner oder CCD-Kameras kann das Verfahren als Kombination von Plasmonenresonanzmikroskopie (Eine ausführliche Beschreibung der Plasmonenresonanzmikroskopie findet sich beispielsweise bei B. Ruthenhäusler et al., Nature, Vol. 332 (1988) 615 bis 617.) und Fluoreszenzdetektion durchgeführt werden. Ein solches Verfahren ermöglicht auch die Analyse von Array-Strukturen, d.h. von mehreren, nebeneinenader angeordneten Reaktionsflächen bzw. -spots auf der Festphase. Eine solche Array-Struktur kann zum simultanen Nachweis mehrerer Analyten in einer Probe oder/und zur simultanen Bestimmung mehrerer Proben eingesetzt werden.

Ein weiterer Gegenstand der Erfindung ist eine Vorrichtung zum Nachweis eines an eine Festphase gebundenen Analyten, welche dadurch gekennzeichnet ist, dass sie Mittel zur Messung der Plasmonenresonanz und der Fluoreszenz und zur unabhängigen Auswertung der Signale aus der Plasmonenresonanz- und der Fluoreszenzmessung umfaßt.

Bevorzugt umfaßt eine solche Vorrichtung einen Laser als Lichtquelle, einen Polarisator zur p-Polarisierung des Laserlichts, einen optisch durchlässigen Träger, der mit einer Metall- oder mit einer Metalloxidschicht überzogen ist, als Festphase, eine Photodiode zur Detektion der Plasmonenresonanz und einen Photomultiplier zur Fluoreszenzdetektion. Die Festphase umfaßt dabei besonders bevorzugt einen Träger, welcher flächig mit einer Metall- oder Metall/Metalloxidschicht überzogen ist, auf der räumlich definierte Reagenzspots aufgetragen sind, die unterschiedl che Festphasenreaktanten oder/und einen Festphasenreaktanten in unterschiedlichen Konzentrationen enthalten. Besonders bevorzugt umfaßt eine solche Vorrichtung Mittel zur Durchführung einer Plasmonenresonanzmikroskopie- und einer Fluoreszenzmessung und zur unabhängigen Auswertung der Signale aus der Plasmonenresonanzmikroskopie- und der Fluoreszenzmessung.

Ein weiterer Gegenstand der Erfindung ist die Verwendung einer solchen Vorrichtung zum Nachweis eines an eine Festphase gebundenen Analyten, z.B. zur Durchführung von Immunoassays oder Nukleinsäure-Hybridisierungsassys. Die Erfindung wird durch die beigefügten Zeichnungen und die folgenden Beispiele weiter veranschaulicht.

Fig. 1    zeigt den experimentellen Aufbau einer erfindungsgemäßen Vorrichtung,

Fig. 2    zeigt Reagenzien, die zur Bildung einer selbstassemblierenden Monoschicht geeignet sind, wobei Fig. 2a ein biotinyliertes Thiol (Festphasenreaktant) und Fig. 2b ein OH-terminiertes Thiol (Verdünnermolekül) zeigt,

Fig. 3    zeigt einen biotinylierten Fluoreszenzmarker und

Fig. 4    zeigt Messungen der Reflektivität und Fluoreszenzintensität, welche mit einer erfindungsgemäßen Vorrichtung erhalten wurden.

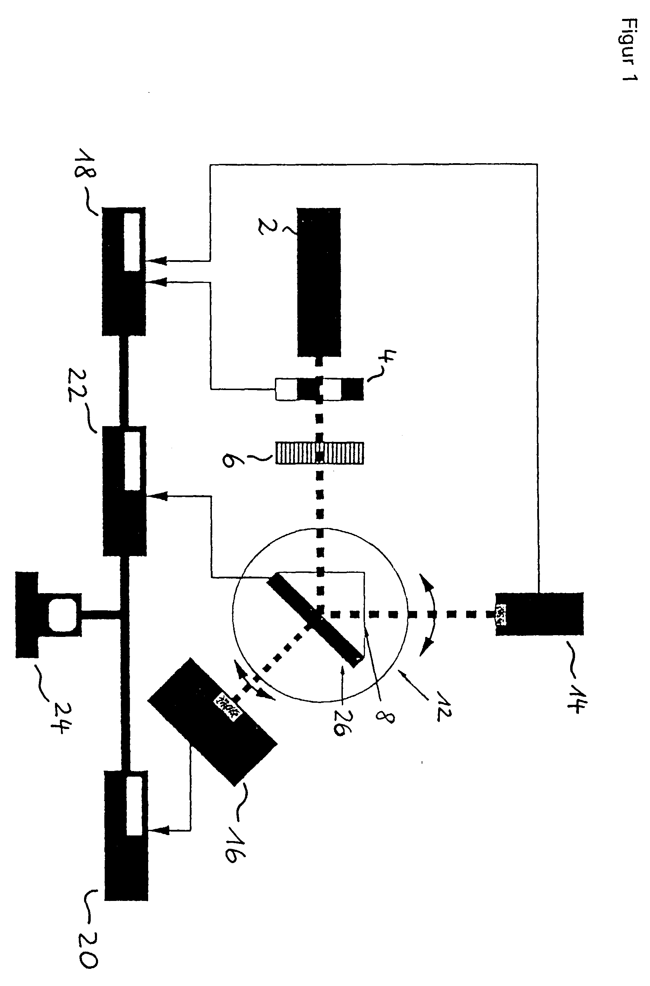

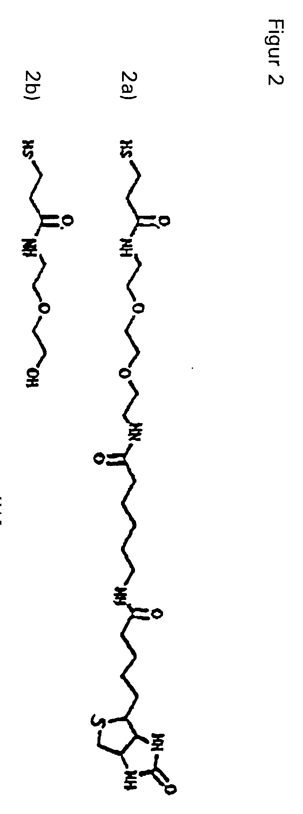

Der Aufbau einer erfindungsgemäßen Vorrichtung ist schematisch in Figur 1 dargestellt und besteht aus einem Laser (2), einem Chopper (4), einem Polarisator (6), einem Prisma (8), einem Goniometer (12), einer Photodiode (14), einem Photomultiplier (16) sowie der entsprechenden Hard- und Software zur Steuerung und Auswertung der Meßergebnisse, umfassend einen Lock In (18), einen Counter (20), einen Temperaturregler (22) sowie einem PC (24). Der in Figur 1 dargestellte Aufbau ermöglicht die simultane winkel- und zeitabhängige Messung der Reflektivität (SPR, Oberflächenplasmonenresonanz) und der Fluoreszenzintensität. Die Anregung der Oberflächenplasmonen in der Metallschicht (26) erfolgt über das eingestrahlte Licht, beispielsweise durch eine Laserdiode mit 650 nm oder einen Laser mit 543 nm. Der kontinuierliche Laserlichtstrahl wird durch den Chopper (4) in zeitlich getrennte Segmente aufgeteilt und durch den Polarisator (6) p-polarisiert. Das p-polarisierte Laserlicht trifft auf ein Glasprisma, an dessen Rückseite sich ein mit einer ca. 50 nm dicken Goldschicht bedampftes Objektträgerglas befindet. Auf der Goldoberfläche sind die Reagenzien, beispielsweise über eine verdünnte selbstassemblierende Monoschicht mit den in Figur 2 dargestellten Reagenzien adsorbiert. Das von der Probe reflektierte Licht (Plasmonenresonanz) wird über die Photodiode (14) und über den Lock In Verstärker (18) gemessen. Parallel dazu wird die Fluoreszenzintensität über den Photomultiplier (16) mit vorgeschaltetem Interferenzfilter und einem Counter (20) ermittelt. Die Steuerung des Experiments und die Messung der Signale erfolgt über einen PC (24).

**Beispiel 1**

Herstellung der Reagenzträger

In einer Hochvakuum-Beschichtungsanlage (Firma Leibold) wird eine ca. 50 nm dicke Goldschicht auf einen Glasträger aus LASFN9 aufgedampft. Unmittelbar nach dem Aufdampfen der Goldschicht wird über einen selbstassemblierenden Adsorptionsprozeß eine Alkanthiolschicht, bestehend aus einer Mischung eines biotinylierten und eines OH-terminierten Thiols wie in Figur 2 dargestellt, aufgebracht. Die Konzentration der Thiole beträgt $10^{-6}$ mol/l in Wasser, wobei der Anteil des biotinylierten Thiol 10%, der Anteil des OH-terminierten Thiols 90% beträgt. Auf diese Schicht wird aus einem PBS-Puffer mit einer Streptavidinkonzentration von 5 x $10^{-6}$ mol/l eine Streptavidinschicht als Monolage auf den Reagenzträger aufgebracht. Ein so hergestellter Träger kann direkt verwendet werden.

**Beispiel 2**

Messung der Adsorption eines biotinylierten Fluoreszenzfarbstoffs

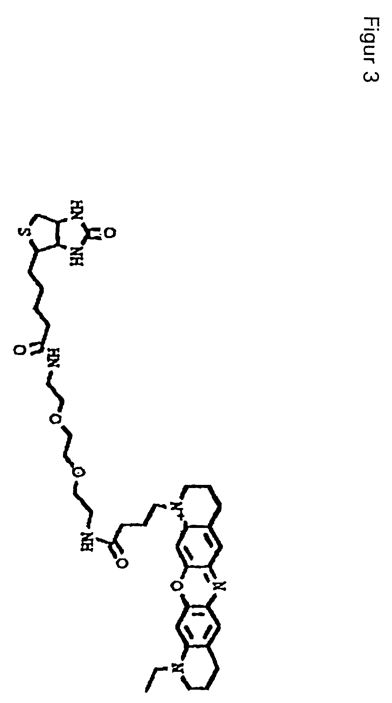

Auf einen wie in Beispiel 2 hergestellten, mit Streptavidin beschichteten Träger wird aus wäßriger Lösung der in Fig. 3 dargestellte biotinylierte Fluoreszenzfarbstoff aufgebracht. Dieser Farbstoff hat ein Absorptionsmaximum bei 464 nm und ein Emissionsmaximum bei 676 nm. Mit der in Beispiel 1 beschriebenen Vorrichtung wurden simultan eine winkelabhängige (Scanbetrieb) und zeitabhängige (Kinetikbetrieb) Messung der Reflektivität und der Fluoreszenzintensität durchgeführt. Die Anregung der Oberflächenplasmonen erfolgte über eine Laserdiode mit einer Wellenlänge mit 650 nm, wobei das einfallende Licht durch einen Polarisator p-polarisiert wurde. Mit der Vorrichtung wurden simultan die Fluoreszenzintensität über einen Photomultiplier sowie die Reflektivität über eine Photodiode ermittelt.

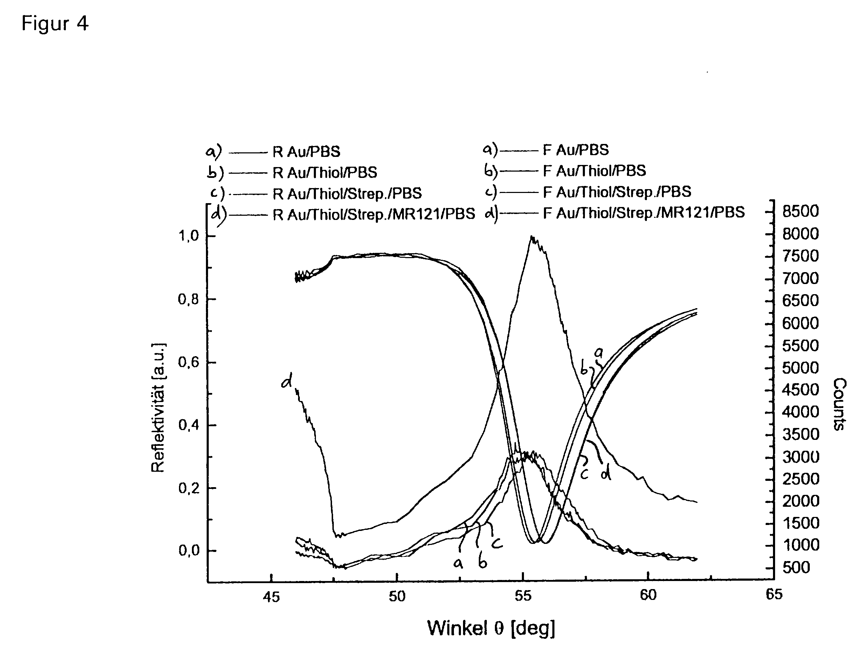

Zunächst wurde eine Alkanthiolschicht auf das Goldsubstrat durch einen selbstorganisierten Adsorptionsprozeß aufgebracht, wie in Beispiel 2 beschrieben. Die Alkanthiolschicht bestand aus einer binären Mischung von biotinylierten Alkanthiolen sowie OH-terminierten Thiolen. Die Verdünnung der biotinylierten Thiole diente der optimalen Exposition der Biotinliganden, um die Bildung der nachfolgend adsorpierten Streptavidinmonoschicht zu begünstigen. Die Konzentration der Thiole betrug $10^{-6}$ mol/l in einem PBS-Puffer (0,137 M NaCl, 0,01 M Phosphatpuffer), wobei 10 Volumenanteile des biotinylierten Thiols und 90% Volumenanteile des OH-terminierten Thiols eingesetzt wurden. Dieses Mischungsverhältnis erlaubte die sterisch unbehinderte Bildung einer Streptavidinmonoschicht aus einer Streptavidinlösung ([SA] = 5 x $10^{-6}$ M) in PBS-Puffer. Nach der Präparation der Streptavidinmatrix wurde eine kombinierte Plasmonenresonanz- Fluoreszenz-Referenzmessung durchgeführt. Diese Messung zeigte bereits eine detektierbare Zunahme der Fluoreszenzintensität im Bereich des Resonanzminimums, die auf die intrinsische Fluoreszenz des Schichtsystems zurückzuführen ist (Fig. 4, Kurven c). Zudem folgte das Intensitätsmaximum der Fluoreszenz, wie durch Fresnellsimulationen theroretisch verhergesagt, der Verschiebung des Resonanzminimums bei Immobilisierung der Thiole und des Streptavidins. Zum Vergleich wurden Messungen an einer Goldoberfläche durchgeführt, auf die lediglich der PBS-Puffer aufgebracht war (Fig. 4, Kurven a) sowie an einer Goldoberfläche, auf die eine selbstassemblierende Monoschicht aus biotinylierten Alkanthiolen sowie OH-terminierten Thiolen, nicht jedoch eine Streptavidinmonoschicht aufgebracht war (Fig. 4, Kurven b).

Schließlich wurde die Adsorption des biotinylierten Farbstoffs durch simultane Fluoreszenz- und Reflektivitätsmessung bestimmt. Hierzu wurde auf eine gemäß Beispiel 2 vorbereitete Oberfläche der biotinylierte Farbstoff adsorbiert. Aus den in Figur 4, Kurven d dargestellten Ergebnissen ist zu erkennen, dass die detektierte Fluoreszenz ein signifikant gegenüber dem Hintergrund erhöhtes Signal zeigt und so die Erkennung der Farbstoffadsorption ermöglicht.

**Patentansprüche**

1. Verfahren zum Nachweis eines Analyten,
   **dadurch gekennzeichnet**,
   daß man die Bindung des Analyten an eine Festphase durch unabhängige Auswertung der Signale aus einer Plasmonenresonanz- und einer Fluoreszenzmessung bestimmt.

2. Verfahren nach Anspruch 1,
   **dadurch gekennzeichnet**,
   daß man einen optisch durchlässigen Träger, der mit einer Metall- oder Metall/Metalloxidschicht überzogen ist, als Festphase verwendet.

3. Verfahren nach Anspruch 2,
   **dadurch gekennzeichnet**,
   daß man einen Träger verwendet, der mit einer Silber- oder Goldschicht überzogen ist.

4. Verfahren nach einem der vorhergehenden Ansprüche,
   **dadurch gekennzeichnet**,
   daß der Analyt über eine Festphasenbindematrix mit der Festphase verknüpft wird.

5. Verfahren nach Anspruch 4,
   **dadurch gekennzeichnet**,
   daß die Festphasenbindematrix eine selbstassemblierende Monoschicht umfaßt.

6. Vorrichtung zum Nachweis eines an eine Festphase gebundenen Analyten,
   **dadurch gekennzeichnet**,
   daß die Vorrichtung Mittel zur Messung der Plasmonenresonanz und der Fluoreszenz und zur unabhängigen Auswertung der Signale aus der Plasmonenresonanz- und der Fluoreszenzmessung umfaßt.

7. Vorrichtung nach Anspruch 6,
   **dadurch gekennzeichnet**,
   daß die Festphase einen Träger umfaßt, welcher flächig mit einer Metall- oder Metall/Metalloxidschicht überzogen ist, auf der mehrere räumlich definierte Reagenzspots angeordnet sind, die unterschiedliche Festphasenreaktanten oder/und einen Festphasenreaktanten in unterschiedlichen Konzentrationen enthalten.

Figur 1

EP 0 872 733 A1

6

Figur 2

2a)

2b)

Figur 3

Figur 4

## EP 0 872 733 A1

| EINSCHLÄGIGE DOKUMENTE | | | EP 98106612.9 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.6) |
|---|---|---|---|
| D,A | EP 0353937 A1 (ARES-SERONO RESEARCH & DEVELOPMENT LIMITED PARTNERSHIP) 07 Februar 1990 (07.02.90), Ansprüche. -- | 1-7 | G 01 N 33/483<br>G 01 N 21/64<br>G 01 N 21/55<br>G 01 N 33/543<br>G 01 N 33/533<br>G 01 N 33/553 |
| A | WO 88/07202 A1 (ARES-SERONO RESEARCH & DEVELOPMENT LIMITED PARTERSHIP) 22 September 1988 (22.09.88), Zusammenfassung, Ansprüche. ---- | 1-7 | |
| | | | RECHERCHIERTE SACHGEBIETE (Int. Cl.6)<br><br>G 01 N |

Der vorliegende Recherchenbericht wurde für alle Patentansprüche erstellt.

| Recherchenort | Abschlußdatum der Recherche | Prüfer |
|---|---|---|
| WIEN | 25-06-1998 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82